(19) Europäisches Patentamt
European Patent Office
Office européen des brevets

(11) Publication number: **0 421 751 A2**

(12) **EUROPEAN PATENT APPLICATION**

(21) Application number: **90310809.0**

(22) Date of filing: **03.10.90**

(51) Int. Cl.5: **C07D 499/32**, C07D 499/46, A61K 31/43

The title of the invention has been amended (Guidelines for Examination in the EPO, A-III, 7.3).

(30) Priority: **05.10.89 GB 8922410**

(43) Date of publication of application: **10.04.91 Bulletin 91/15**

(84) Designated Contracting States: **CH DE FR GB IT LI NL**

(71) Applicant: **Beecham Group p.l.c.**
**SB House Great West Road**
**Brentford Middlesex TW8 9BD(GB)**

(72) Inventor: **Ponsford, Roger John**
**SmithKline Beecham Pharmaceutic.,**
**Brockham Park**
**Betchworth, Surrey RH3 7AJ(GB)**
Inventor: **Stachulski, Andrew Valentine**
**SmithKline Beecham Pharmaceutic.,**
**Brockham Park**
**Betchworth, Surrey RH3 7AJ(GB)**

(74) Representative: **West, Vivien et al**
**SmithKline Beecham, Corporate Patents,**
**Great Burgh, Yew Tree Bottom Road**
**Epsom, Surrey KT18 5XQ(GB)**

(54) **Substituted acrylamido-penicillanic acid esters.**

(57) Compounds of formula (I):

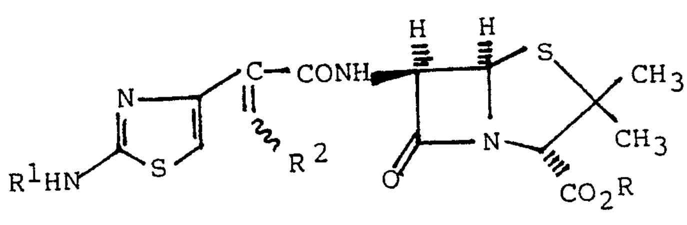

(I)

wherein $R^1$ is hydrogen or an amino protecting group, $R^2$ is a hydrogen atom, an optionally substituted alkyl, alkenyl, alkynyl or cycloalkyl group, aryl or an optionally substituted heterocyclyl group, and R is a group $CHR^a$ $OCOR^b$, wherein $R^a$ is hydrogen, $C_{1-6}$ alkyl, $C_{3-7}$ cycloalkyl, methyl or phenyl, and $R^b$ is $C_{1-6}$ alkyl, $C_{1-6}$ alkoxy, phenyl, optionally substituted with -NH $COCHR^cNH_2$, benzyl, $C_{3-7}$ cycloalkyl, or $C_{1-6}$ alkyl $C_{3-7}$ cycloalkyl; or $R^a$ and $R^b$ together form a 1,2-phenylene group optionally substituted by one or two methoxy groups, wherein $R^c$ represents hydrogen or $C_{1-6}$ alkyl, are useful in the treatment of bacterial infections.

EP 0 421 751 A2

## NOVEL COMPOUNDS

This invention relates to novel β-lactam containing compounds, their preparation and their use, and in particular to a novel class of penicillins. These compounds have antibacterial properties, and therefore are of use in the treatment of bacterial infections in humans and animals caused by a wide range of organisms.

US-A-3,622,569, US-A-4,416,880, US-A-4,782,162, US-A-4,500,716, GB-A-2,173,194 and JP-A-2,215,593 disclose β-lactam antibiotics containing a substituted acrylamido side chain.

The present invention provides a compound of formula (I):

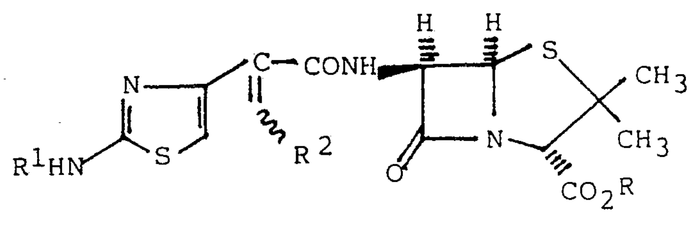

(I)

wherein $R^1$ is hydrogen or an amino protecting group, $R^2$ is a hydrogen atom, an optionally substituted alkyl, alkenyl, alkynyl or cycloalkyl group, aryl or an optionally substituted heterocyclyl group, and R is a group $CHR^a OCOR^b$, wherein $R^a$ is hydrogen, $C_{1-6}$ alkyl, $C_{3-7}$ cycloalkyl, methyl or phenyl, and $R^b$ is $C_{1-6}$ alkyl, $C_{1-6}$ alkoxy, phenyl, optionally substituted with -NH $COCHR^cNH_2$, benzyl, $C_{3-7}$ cycloalkyl, or $C_{1-6}$ alkyl $C_{3-7}$ cycloalkyl; or $R^a$ and $R^b$ together form a 1,2-phenylene group optionally substituted by one or two methoxy groups, wherein $R^c$ represents hydrogen or $C_{1-6}$ alkyl.

Examples of suitable groups R include, for example, acyloxyalkyl groups such as acetoxymethyl, pivaloyloxymethyll α-acetoxyethyl, α-pivaloyloxyethyl, and 1-(cyclohexylcarbonyloxy)prop-1-yl; alkoxycarbonyloxyalkyl groups, such as ethoxycarbonyloxymethyl and α-ethoxycarbonyl oxyethyl; and lactone groups such as phthalidyl and dimethoxyphthalidyl.

In particular, esters of compounds of formula (I) have been found to give high levels of oral adsorption.

When $R^2$ is optionally substituted alkyl, alkenyl or alkynyl, it may be straight or branched chain and may contain, for example, up to 18, more preferably up to 12, carbon atoms, suitably from 1 to 6 carbon atoms. In particular the group may be substituted by double bonded oxygen, nitrogen or sulphur or by hydroxy, aryl, $C_{1-6}$ alkoxy, amino, mono- or di- $C_{1-6}$ alkylamino, methylene optionally substituted by halogen, halogen, or trifluoromethyl.

When $R^2$ is cycloalkyl it contains up to 18 carbon atoms, preferably 5 to 7 carbon atoms, and is optionally substituted as set out hereinabove for alkyl.

When used herein the term 'aryl' includes optionally substituted phenyl and polycyclic aromatic rings, and in particular it includes phenyl and naphthyl optionally substituted with up to five, preferably up to three, groups selected from halogen, $C_{1-6}$ alkyl, phenyl, $C_{1-6}$ alkoxy, halo($C_{1-6}$) alkyl, hydroxy, amino, nitro, carboxy, $C_{1-6}$ alkoxycarbonyl, $C_{1-6}$ alkoxycarbonyl-($C_{1-6}$)-alkyl, $C_{1-6}$ alkylcarbonyloxy, or $C_{1-6}$ alkylcarbonyl groups.

The term 'heterocyclyl' includes single or fused rings comprising up to four hetero atoms in the ring selected from oxygen, nitrogen and sulphur and optionally substituted with up to three halogen, $C_{1-6}$ alkyl, $C_{1-6}$ alkoxy, halo-($C_{1-6}$)-alkyl, hydroxy, amino, carboxy, $C_{1-6}$ alkoxycarbonyl, $C_{1-6}$ alkoxycarbonyl($C_{1-6}$) alkyl, aryl or oxo groups.

Suitably the heterocyclic ring comprises from 4 to 7 ring atoms, preferably 5 to 6 atoms.

The term 'halogen' refers to fluorine, chlorine, bromine and iodine.

Preferably $R^2$ is an unsubstituted or substituted phenyl.

Compounds of the invention may exist in two or more tautomeric forms, e.g. those having the partial structures below:

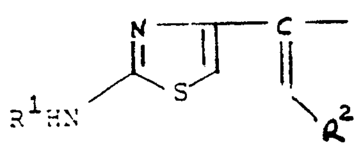
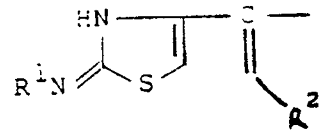

It should be understood that all tautomeric forms of the compound of formula (I) are included within the scope of the invention.

Suitable amino protecting groups $R^1$ are those well known in the art which may be removed under conventional conditions without disruption of the remainder of the molecule.

Examples of amino protecting groups $R^1$ include $C_{1-6}$ alkanoyl; benzoyl or benzyl optionally substituted in the phenyl ring by one or two substituents selected from $C_{1-4}$ alkyl, $C_{1-4}$ alkoxy, trifluoromethyl, halogen, or nitro; $C_{1-4}$ alkoxycarbonyl; benzyloxycarbonyl or trityl substituted as for benzyl above; allyloxycarbonyl, trichloroethoxycarbonyl or chloroacetyl.

Some of the compounds of this invention may be crystallised or recrystallised from solvents such as methanol. In such cases solvates may be formed. This invention includes within its scope stoichiometric solvates including hydrates as well as compounds containing variable amounts of water that may be produced by processes such as lyophilisation.

Since the compounds of the formula (I) are intended for use in pharmaceutical compositions it will readily be understood that they are each provided in substantially pure form, for example at least 60% pure, more suitably at least 75% pure and preferably at least 85%, especially at least 95% pure (% are on a weight for weight basis). Impure preparations of the compounds of the formula (I) and their salts may be used for preparing the more pure forms used in the pharmaceutical compositions; these less pure preparations of the compounds of formula (I) and their salts should contain at least 1%, more suitably at least 5% and preferably from 10 to 49% of a compound of the formula (I) or salt thereof.

Compounds of the present invention may exist as either syn or anti isomers, or may exist as mixtures of syn and anti isomers containing at least 75% of one such isomer, or preferably at least 90% of one such isomer.

Herein the terms syn and anti refer to the configuration of the group $R^2$ with respect to the carboxamido group, the syn -configuration (sometimes called the Z-configuration) being denoted thus:

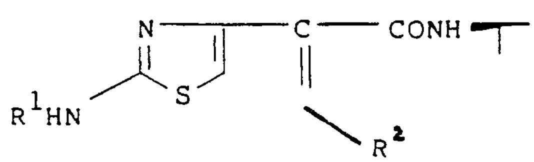

and the anti configuration (sometimes called the E-configuration) being denoted thus:

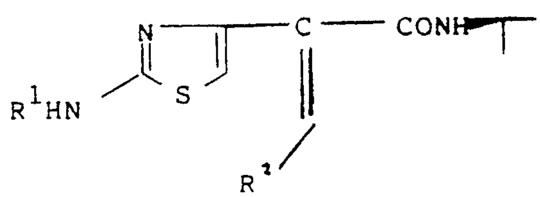

Preferred compounds of the present invention are the syn -isomers of the formula (IA):

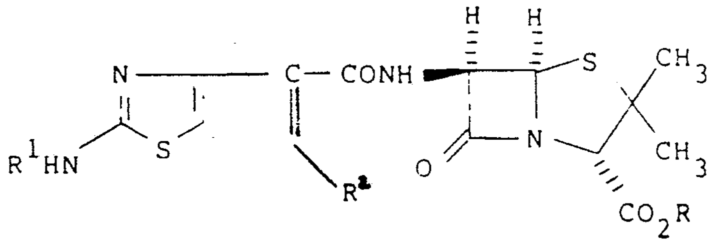

(IA)

wherein R, $R^1$ and $R^2$ are as hereinbefore defined.

The compounds of formula (I) may be prepared by treating a compound of formula (II) or a salt thereof:

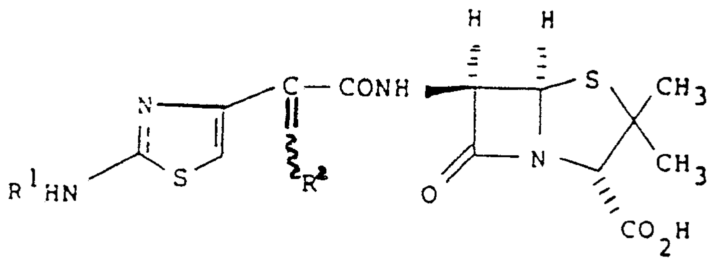

(II)

wherein $R^1$ and $R^2$ are as hereinbefore defined, with a compound of the formula $XCHR^aOCOR^b$ in which $R^a$ and $R^b$ are as hereinbefore defined and X is a leaving group.

Examples of suitable leaving groups X include halogen as hereinbefore defined and a particularly suitable example is bromine.

Suitable salts of the compound of formula (II) include sodium, potassium, silver, and amine salts e.g. triethylamine, diisopropylethylamine and the like.

The reaction may be carried out at non-extreme temperature in conventional solvents such as dimethylformamide, dimethyl sulphoxide, acetonitrile, and the like.

The preferred compound of formula (I) may be prepared by esterifying 6β-[Z-[2-(2-aminothiazol-4-yl)-3-phenyl]-propenamido]penicillanic acid or a salt thereof with 1-bromoethyl acetate in anhydrous dimethylformamide at a temperature of 0°C.

The free acid starting material may be prepared according to the process described in Example 32 of US-A-4416880.

The present invention also provides a pharmaceutical composition which comprises a compound of formula (I) and a pharmaceutically acceptable carrier. The compositions of the invention include those in a form adapted for oral, topical or parenteral use and may be used for the treatment of the infection in mammals including humans.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The composition may be formulated for administration by any route, such as oral, topical or parenteral. The compositions may be in the form of tablets, capsules, powders, granules, lozenges, creams or liquid preparations, such as oral or sterile parenteral solutions or suspensions. Administration by the oral route is preferred.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone: fillers, for example lactose, sugar, maize-starch, calcium phosphate, sorbitol or glycine,

tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for iniection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for iniection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the composition comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) is administered in the above-mentioned dosage range.

The compound of the invention of formula (I) may be used as the sole therapeutic agent in compositions of the invention or may be used in combination with other antibiotics or with a $\beta$-lactamase inhibitor.

Advantageously the compositions also comprise a compound of formula (III) or a pharmaceutically acceptable salt or ester thereof:

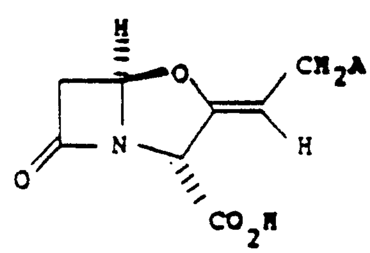

(III)

wherein A is hydroxyl; substituted hydroxyl; thiol; a group of formula $SO_2R^5$ wherein $R^5$ is $C_{1-6}$ alkyl; substituted thiol; amino; mono- or di-hydrocarbyl substituted amino; mono- or di-acylamino; an optionally substituted triazolyl group; or an optionally substituted tetrazolyl group as described in EP O 053 893.

A further advantageous composition comprises an antibiotic compound according to the invention and a pharmaceutically acceptable carrier or excipient together with a $\beta$-lactamase inhibitor of formula (IV) or a pharmaceutically acceptable salt or *in vivo* hydrolysable ester thereof:

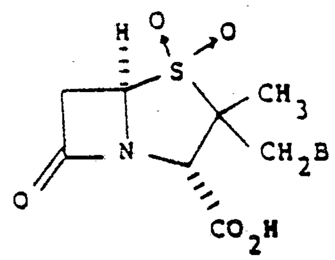

(IV)

wherein B is hydrogen, halogen or a group of formula:

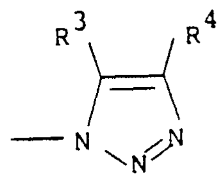

in which $R^3$ and $R^4$ are the same or different and each is hydrogen, $C_{1-6}$ alkoxycarbonyl, or carboxy or a pharmaceutically acceptable salt thereof.

Further suitable β-lactamase inhibitors include 6-alkylidene penems of formula (V) below:

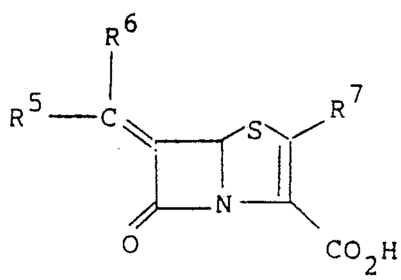

(V)

or a pharmaceutically acceptable salt or in vivo hydrolysable ester thereof, wherein $R^5$ and $R^6$ are the same or different and each represents hydrogen, or a $C_{1-10}$ hydrocarbon or heterocyclic group optionally substituted with a functional group; and $R^7$ represents hydrogen or a group of formula $R^d$ or $-SR^d$ where $R^d$ is an optionally substituted $C_{1-10}$ hydrocarbon or heterocyclic group, as described in EP-A-0 041 768.

Other suitable β-lactamase inhibitors include 6β-bromopenicillanic acid and salts and in vivo hydrolysable esters thereof and 6β-iodopenicillanic acid and salts and in vivo hydrolysable esters thereof.

Such compositions of this invention comprising a β-lactamase inhibitor are formulated in conventional manner.

The present invention also includes a method of treating bacterial infections in humans and animals which comprises the administration of a therapeutically effective amount of an antibiotic compound of this invention.

Antibiotic compounds of the present invention are active against a broad range of bacteria, in particular they are useful for treatment of respiratory tract and urinary tract infections in humans and mastitis in cattle. It should be stressed that a particular advantage of certain compounds of the invention is their stability to β-lactamase enzymes and they are therefore effective against β-lactamase-producing organisms.

The following Example illustrates the preparation of a compound of the present invention:

Example 1

1-Acetoxyethyl 6β-[Z-[2-(2-aminothiazol-4-yl)-3-phenyl]propenamido]penicillanate

6β-[Z-[2-(2-Aminothiazol-4-yl)-3-phenyl]propenamido]penicillanic acid (0.422g) in anhydrous dimethylformamide (3ml) was treated with triethylamine (0.265ml) and then stirred at 0°C whilst 1-bromoethyl acetate (0.238g) was added. The mixture was allowed to regain ambient temperature and stirred for 2.5h, after which time reaction appeared complete by t.l.c. analysis. The mixture was diluted with ethyl acetate (15ml) and washed sequentially with 5% citric acid (3ml), brine (3ml), saturated aqueous sodium hydrogen carbonate (3ml) and again with brine (3x3ml). The organic phase was then dried and evaporated to a yellow oil, which was twice chromatographed on silica gel, eluting with ethyl acetate:hexane, 1:1. Appropriate fractions were pooled and evaporated to a yellow gum, which solidified on azeotroping with dichloromethane to give the required product (0.177g); $\nu_{max}$ (KBr) 1790(sh), 1765, 1670, 1610, 1530, and 1500-(sh)cm$^{-1}$; $\delta$[$(CD_3)_2CO$,250MHz], inter alia , 1.44-1.58 (6H,m), 4.35 (1H,2s), 5.67, 5.85 (2H,2m), 6.50 (2H,br s,$D_2O$ exch.), 6.55, 6.57 (1H,2s), 6.83-6.96 (1H,m), 7.23-7.53 (6H,m), and 7.90-8.00 (1H,m,$D_2O$ exch.); m/e 531 ($MH^+$).

## Claims

1. A compound of formula (I):

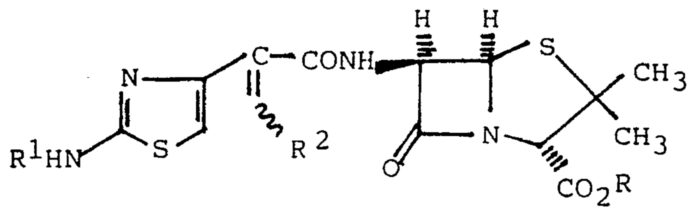

(I)

wherein $R^1$ is hydrogen or an amino protecting group, $R^2$ is a hydrogen atom, an optionally substituted alkyl, alkenyl, alkynyl or cycloalkyl group, aryl or an optionally substituted heterocyclyl group, and R is a group $CHR^a$ $OCOR^b$, wherein $R^a$ is hydrogen, $C_{1-6}$ alkyl, $C_{3-7}$ cycloalkyl, methyl or phenyl, and $R^b$ is $C_{1-6}$ alkyl, $C_{1-6}$ alkoxy, phenyl, optionally substituted with -NH $COCHR^cNH_2$, benzyl, $C_{3-7}$ cycloalkyl, or $C_{1-6}$ alkyl $C_{3-7}$ cycloalkyl; or $R^a$ and $R^b$ together form a 1,2-phenylene group optionally substituted by one or two methoxy groups, wherein $R^c$ represents hydrogen or $C_{1-6}$ alkyl.

2. A compound according to claim 1, wherein R is acyloxyalkyl, alkoxycarbonyloxyalkyl, or a lactone group.

3. A compound according to claim 1 or 2, wherein $R^2$ is optionally substituted phenyl.

4. A compound according to any preceding claim, which is in the form of the syn isomer.

5. 1-Acetoxyethyl 6β-[Z-[2-(2-aminothiazol-4-yl)-3-phenyl]propenamido]penicillanate.

6. A process for the preparation of a compound according to any preceding claim, which process comprises treating a compound of formula (II) or a salt thereof:

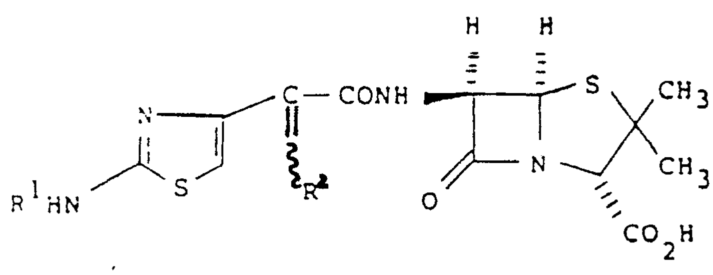

(II)

wherein $R^1$ and $R^2$ are as hereinbefore defined, with a compound of the formula $XCHR^aOCOR^b$ in which $R^a$ and $R^b$ are as hereinbefore defined and X is a leaving group.

7. A pharmaceutical composition which comprises a compound according to any one of claims 1 to 5 and a pharmaceutically acceptable carrier.

8. Use of a compound according to any one of claims 1 to 5 for the treatment of bacterial infections in humans and animals, or in the manufacture of a composition according to claim 7.